# EUROPÄISCHE PATENTSCHRIFT

(11) **EP 1 432 805 B1**
(45) Veröffentlichungstag und Bekanntmachung des Hinweises auf die Patenterteilung: **18.05.2005**
(21) Anmeldenummer: 02781133.0
(22) Anmeldetag: 02.10.2002
(51) Int. Cl.: C12N 15/30

(54) **DNA-EXPRESSIONSKONSTRUKT ZUR BEHANDLUNG VON INFEKTIONEN MIT LEISHMANIOSE**
DNA-EXPRESSION CONSTRUCT FOR TREATMENT OF INFECTIONS WITH LEISHMANIASIS
CONSTRUCTION D'EXPRESSION D'ADN DESTINEE AU TRAITEMENT D'INFECTIONS DE TYPE LEISHMANIOSE

(30) Priorität: 02.10.2001 DE 10148732; 12.11.2001 DE 10156679
(43) Veröffentlichungstag der Anmeldung: 30.06.2004
(73) Patentinhaber: Mologen AG, 14195 Berlin (DE)
(72) Erfinder: FUERTES, Laura, Lopez, 28003 Madrid (ES); TIMON, JIMENEZ, Marcos, 28200 San Lorenzo de El Escorial (ES)
(74) Vertreter: Bergmann, Simon
(86) Internationale Anmeldenummer: PCT/DE2002/003799
(87) Internationale Veröffentlichungsnummer: WO 2003/031470

(56) Entgegenhaltungen:
- WO-A-98/21322
- GONZALEZ-ASEGUINOLAZA GLORIA ET AL: "Molecular cloning, cell localization and binding affinity to DNA replication proteins of the p36/LACK protective from Leishmania infantum." EUROPEAN JOURNAL OF BIOCHEMISTRY, Bd. 259, Nr. 3, Februar 1999 (1999-02), Seiten 909-916, XP002246229 ISSN: 0014-2956 in der Anmeldung erwähnt
- MARIA GONZALO ROSA ET AL: "Protective immune response against cutaneous leishmaniasis by prime/booster immunization regimens with vaccinia virus recombinants expressing Leishmania infantum p36/LACK and IL-12 in combination with purified p36." MICROBES AND INFECTION, Bd. 3, Nr. 9, Juli 2001 (2001-07), Seiten 701-711, XP002246230 ISSN: 1286-4579 in der Anmeldung erwähnt
- LANFORD R E ET AL: "INDUCTION OF NUCLEAR TRANSPORT WITH A SYNTHETIC PEPTIDE HOMOLOGOUS TO THE SV-40 T ANTIGEN TRANSPORT SIGNAL" CELL, Bd. 46, Nr. 4, 1986, Seiten 575-582, XP009013265 ISSN: 0092-8674 in der Anmeldung erwähnt

### SEQUENCE LISTING

<110> Mologen Forschungs-, Entwicklungs- und Vertriebs GmbH
<120> DNA-EXPRESSION CONSTRUCT FOR TREATMENT OF INFECTIONS WITH LEISHMANIASIS
<130> XI 1396/02
<140> PCT/ DE 02/ 03799
   <141> 2002-10-02
<150> DE 101 48 732.0
   <151> 2001-10-02
<150> DE 101 56 679.4
   <151> 2001-11-12
<160> 9
<170> PatentIn version 3.1
<210> 1
   <211> 4791
   <212> DNA
   <213> artificial sequence
<220>
   <223> Plasmid pSCp36
<220>
   <221> misc_feature
   <222> (1939)..(1045)
   <223> Kanamycin resistance, reverse complementary
<220>
   <221> promoter
   <222> (2447)..(3243)
   <223> CMV
<220>
   <221> Intron
   <222> (3250)..(3386)
   <223>
<220>
   <221> misc_feature
   <222> (3393)..(4331)
   <223> p36 protein
<220>
   <221> polyA_site
   <222> (4338)..(4539)
   <223> poly A site aus SV 40
<400> 1
   tcttccgctt cctcgctcac tgactcgctg cgctcggtcg ttcggctgcg gcgagcggta     60
   tcagctcact caaaggcggt aatacggtta tccacagaat caggggataa cgcaggaaag    120
   aacatgtctc gggaggcctc acgtgacatg tgagcaaaag gccagcaaaa ggccaggaac 180
   cgtaaaaagg ccgcgttgct ggcgtttttc cataggctcc gcccccctga cgagcatcac 240
   aaaaatcgac gctcaagtca gaggtggcga aacccgacag gactataaag ataccaggcg 300
   tttccccctg gaagctccct cgtgcgctct cctgttccga ccctgccgct taccggatac 360
   ctgtccgcct ttctcccttc gggaagcgtg gcgctttctc atagctcacg ctgtaggtat 420
   ctcagttcgg tgtaggtcgt tcgctccaag ctgggctgtg tgcacgaacc ccccgttcag 480
   cccgaccgct gcgccttatc cggtaactat cgtcttgagt ccaacccggt aagacacgac 540
   ttatcgccac tggcagcagc cactggtaac aggattagca gagcgaggta tgtaggcggt 600
   gctacagagt tcttgaagtg gtggcctaac tacggctaca ctagaaggac agtatttggt 660
   atctgcgctc tgctgaagcc agttaccttc ggaaaaagag ttggtagctc ttgatccggc 720
   aaacaaacca ccgctggtag cggtggtttt tttgtttgca agcagcagat tacgcgcaga 780
   aaaaaaggat ctcaagaaga tcctttgatc ttttctacgg ggtctgacgc tcagtggaac 840
   gaaaactcac gttaagggat tttggtcatg agattatcaa aaaggatctt cacctagatc 900
   cttttaaatt aaaaatgaag ttttaaatca atctaaagta tatatgagta aacttggtct 960
   gacagttacc aatgcttaat cagtgaggca cctatctcag cgatctgtct atttcgttca 1020
   tccatagttg cctgactccc cgtctcagaa gaactcgtca agaaggcgat agaaggcgat 1080
   gcgctgcgaa tcgggagcgg cgataccgta aagcacgagg aagcggtcag cccattcgcc 1140
   gccaagctct tcagcaatat cacgggtagc caacgctatg tcctgatagc ggtccgccac 1200
   acccagccgg ccacagtcga tgaatccaga aaagcggcca ttttccacca tgatattcgg 1260
   caagcaggca tcgccatggg tcacgacgag atcctcgccg tcgggcatgc tcgccttgag 1320
   cctggcgaac agttcggctg gcgcgagccc ctgatgctct tcgtccagat catcctgatc 1380
   gacaagaccg gcttccatcc gagtacgtgc tcgctcgatg cgatgtttcg cttggtggtc 1440
   gaatgggcag gtagccggat caagcgtatg cagccgccgc attgcatcag ccatgatgga 1500
   tactttctcg gcaggagcaa ggtgagatga caggagatcc tgccccggca cttcgcccaa 1560
   tagcagccag tcccttcccg cttcagtgac aacgtcgagc acagctgcgc aaggaacgcc 1620
   cgtcgtggcc agccacgata gccgcgctgc ctcgtcttgc agttcattca gggcaccgga 1680
   caggtcggtc ttgacaaaaa gaaccgggcg cccctgcgct gacagccgga acacggcggc 1740
   atcagagcag ccgattgtct gttgtgccca gtcatagccg aatagcctct ccacccaagc 1800
   ggccggagaa cctgcgtgca atccatcttg ttcaatcata atattattga agcatttatc 1860
   agggttattg tctcatgagc ggatacatat ttgaatgtat ttagaaaaat aaacaaatag 1920
   gggttccgcg cacatttccc cgaaaagtgc cacctgacgt ctaagaaacc attattatca 1980
   tgacattaac ctataaaaat aggcgtatca cgaggccctt tcgtctcgcg cgtttcggtg 2040
   atgacggtga aaacctctga cacatgcagc tcccggagac ggtcacagct tgtctgtaag 2100
   cggatgccgg gagcagacaa gcccgtcagg gcgcgtcagc gggtgttggc gggtgtcggg 2160
   gctggcttaa ctatgcggca tcagagcaga ttgtactgag agtgcaccat atgcggtgtg 2220
   aaataccgca cagatgcgta aggagaaaat accgcatcag gcgccattcg ccattcaggc 2280
   tgcgcaactg ttgggaaggg cgatcggtgc gggcctcttc gctattacgc cagctggcga 2340
   aagggggatg tgctgcaagg cgattaagtt gggtaacgcc agggttttcc cagtcacgac 2400
   gttgtaaaac gacggccagt gccaagcttg gtctcctccc ggatcctcaa tattggccat 2460
   tagccatatt attcattggt tatatagcat aaatcaatat tggctattgg ccattgcata 2520
   cgttgtatct atatcataat atgtacattt atattggctc atgtccaata tgaccgccat 2580
   gttggcattg attattgact agttattaat agtaatcaat tacggggtca ttagttcata 2640
   gcccatatat ggagttccgc gttacataac ttacggtaaa tggcccgcct ggctgaccgc 2700
   ccaacgaccc ccgcccattg acgtcaataa tgacgtatgt tcccatagta acgccaatag 2760
   ggactttcca ttgacgtcaa tgggtggagt atttacggta aactgcccac ttggcagtac 2820
   atcaagtgta tcatatgcca agtccgcccc ctattgacgt caatgacggt aaatggcccg 2880
   cctggcatta tgcccagtac atgaccttac gggactttcc tacttggcag tacatctacg 2940
   tattagtcat cgctattacc atggtgatgc ggttttggca gtacaccaat gggcgtggat 3000
   agcggtttga ctcacgggga tttccaagtc tccaccccat tgacgtcaat gggagtttgt 3060
   tttggcacca aaatcaacgg gactttccaa aatgtcgtaa taaccccgcc ccgttgacgc 3120
   aaatgggcgg taggcgtgta cggtgggagg tctatataag cagaggtcgt ttagtgaacc 3180
   gtcagatcac tagaagcttt attgcggtag tttatcacag ttaaattgct aacgcagtca 3240
   gtgctcgagc aggtaagtat caaggttaca agacaggttt aaggaggcca atagaaactg 3300
   ggcttgtcga gacagagaag actcttgcgt ttctgatagg cacctattgg tcttactgac 3360
   atccactttg cctttctctc cacaggggta ccatgaacta cgagggtcac ctgaagggcc 3420
   accgcggatg ggtcacctcc ctggcctgcc cgcagcaggc ggggtcgtac atcaaggtgg 3480
   tgtcgacgtc gcgcgatggc acggccatct cgtggaaagc caaccccgac cgccacagcg 3540
   tggacagcga ctacggtctg ccgagccacc gcctcgaggg ccacaccggc ttcgtgtcgt 3600
   gtgtgtcgct ggcccacgcc accgactacg cgctgaccgc gtcctgggac cgctccatcc 3660
   gcatgtggga cctgcgcaat ggccagtgcc agcgcaagtt cctgaagcac accaaggacg 3720
   tgctcgccgt cgccttctcg ccggacgacc gcctgatcgt gtccgcgggc cgcgacaacg 3780
   tgatccgcgt gtggaacgtg gcgggcgagt gcatgcacga gttcctgcgc gacggccacg 3840
   aggactgggt gagcagcatc tgtttctcgc cgtcgctgga gcatccgatc gtggtgtccg 3900
   gcagctggga caacaccatc aaggtatgga acgtgaacgg gggcaagtgt gagcgcacgc   3960
   tcaagggcca cagcaactac gtgtccacgg tgacggtgtc gccagacggg tcgctgtgcg   4020
   cgtccggcgg caaggacggc gcggcgctgc tgtgggacct gagcaccggc gagcagctgt   4080
   tcaagatcaa cgtggagtcg cccatcaacc agatcgcctt ctcgcccaac cgcttctgga   4140
   tgtgcgtcgc gacggagagg tccctgtccg tgtacgacct ggagagcaag gctgtgattg   4200
   cggagctgac gccggacggc gcgaagccgt ccgagtgcat ctccattgcc tggtccgccg   4260
   acggcaacac tctgtactcc ggtcacaagg acaacctgat ccgcgtgtgg tccatctccg   4320
   acgccgagta agagctcgat gagtttggac aaaccacaac tagaatgcag tgaaaaaaat   4380
   gctttatttg tgaaatttgt gatgctattg ctttatttgt aaccattata agctgcaata   4440
   aacaagttaa caacaacaat tgcattcatt ttatgtttca ggttcagggg gaggtgtggg   4500
   aggtttttta aagcaagtaa aacctctaca aatgtggtag aattcagggg gagacccaat   4560
   tcgtaatcat ggtcatagct gtttcctgtg tgaaattgtt atccgctcac aattccacac   4620
   aacatacgag ccggaagcat aaagtgtaaa gcctggggtg cctaatgagt gagctaactc   4680
   acattaattg cgttgcgctc actgcccgct ttccagtcgg gaaacctgtc gtgccagctg   4740
   cattaatgaa tcggccaacg cgcggggaga ggcggtttgc gtattgggcg c            4791
<210> 2
   <211> 11
   <212> PRT
   <213> Human immunodeficiency virus type 1
<220>
   <221> MISC_FEATURE
   <223> TAT peptide
<400> 2
   Tyr Gly Arg Lys Lys Arg Arg Gln Arg Arg Arg
   1               5                   10
<210> 3
   <211> 7
   <212> PRT
   <213> Simian virus 40
<220>
   <221> MISC_FEATURE
   <223> NLS peptide
<400> 3
   Pro Lys Lys Lys Arg Lys Val
   1 5
<210> 4
   <211> 20
   <212> DNA
   <213> artificial sequence
<220>
   <223> ODN 1
<220>
   <221> modified_base
   <222> (12)..(12)
   <223> xT = Thymin modified with a reactive amino group
<400> 4
   gggagtccag ttttctggac 20
<210> 5
   <211> 20
   <212> DNA
   <213> artificial sequence
<220>
   <223> ODN 2
<400> 5
   aggggtccag ttttctggac 20
<210> 6
   <211> 33
   <212> DNA
   <213> artificial sequence
<220>
   <223> 1. PCR: Primer left
<400> 6
   ttatatggta ccatgaacat acgagggtca cct 33
<210> 7
   <211> 42
   <212> DNA
   <213> artificial sequence
<220>
   <223> 1. PCR: Primer right
<400> 7
   ttatatgagc tcagaagaca cggacaggga cctcttccgt cg                    42
<210> 8
   <211> 33
   <212> DNA
   <213> artificial sequence
<220>
   <223> 2. PCR: Primer left
<400> 8
   ttatatggta ccatgaacat acgagggtca cct                               33
<210> 9
   <211> 33
   <212> DNA
   <213> artificial sequence
<220>
   <223> 2. PCR: Primer right
<400> 9
   ttatatgagc tcttactcgg ccgtcggaga tgg                               33

## Beschreibung

Die Erfindung betrifft ein DNA-Expressionskonstrukt zur Behandlung von Leishmaniose-Infektionen, sowie eine entsprechende Vakzine.

Leishmanien sind trypanosomatide Flagellaten aus der Ordnung Kinetoplastida, die durch weibliche blutsaugende Sandmücken der Gattung Phlebotomus und Lutzomyia auf verschiedene Säugetierspezies und auf den Menschen übertragen werden. Leishmaniosen sind Erkrankungen mit verschiedenen klinischen Krankheitsbildern, die ein bedeutendes Gesundheitsproblem darstellen. Die WHO schätzt, dass weltweit ca. 12 Millionen Menschen von der Krankheit betroffen sind. Ca. 2 bis 9% aller HIV-Patienten erkranken an viceraler Leishmaniose, damit ist dies die dritt häufigste parasitäre Krankheit, an der HIV-positive Personen erkranken.

Chemotherapie als Behandlungsmethode zeigt nur einen geringen Effekt.

Da Personen, die die Infektion überstanden haben, eine starke Immunität gegen eine spätere Infektion entwickeln, sollte die Entwicklung eines wirksamen Impfschutzes möglich sein.

Das Prinzip der Immunisierung beruht auf der Wiedererkennung von Strukturen bereits erfolgreich bekämpfter Erreger. Zwei Hauptarme der Immunabwehr können dabei unterschieden werden: der humorale, welcher auf der Synthese von Antikörpem beruht, die in der Lage sind, im extrazellulären Raum befindliche Bakterien zu bekämpfen und der zelluläre, der im Wesentlichen auf der Aktivität von T-Lymphozyten des Immunsystems beruht. T-Lymphozyten sind in der Lage, mit Viren infizierte Zellen zu erkennen. Die humorale Immunabwehr wird auch als Th2 pathway und die zelluläre Immunabwehr als Th1 pathway bezeichnet. Die Impfung bzw. Immuntherapie von Leishmaniose, deren Ursache intrazelluläre Parasiten sind, sollte durch die Erzeugung einer Th1-typischen Immunreaktion möglich sein. Im Stand der Technik wird vielfach auf die Bedeutung der Erzeugung einer Th1 Antwort in der Therapie oder Prävention von Leishmaniose hingewiesen. (Handman et al., J Immunol 160: 3949-57, Gurunathan et al., Nature Med: 4( 12): 1409-15). Zur Förderung der Auslösung einer Th1-typischen Immunantwort wird auf das kostimulatorische Zytokin IL-12 als unerlässliches Adjuvanz verwiesen (Parker et al., J. Immunol. 140: 896-902).

Verschiedene Antigene wurden in experimentellen Impfprotokollen in Mäusen getestet. BALB/c Mäuse sind ein gutes Modell zum Studium der Leishmaniose. Es bestehen im Infektionsverlauf und der Läsionsentwicklung sehr große Ähnlichkeiten zum Menschen. Die immunologische Reaktion in Mäusen auf diese Infektion, scheint der im Menschen und wahrscheinlich auch der in Hunden zu gleichen (Cox, Int. J. Parasitol. 263: 1147-1157). Verwendete Antigene waren das gp 63 (Scott et al., J. Exp Med.168: 1675-1684), gp 46 (McMahon-Pratt et al., Infection and Immunity 61: 3351-3359), p-4 und p-8 (Scott et al., Immunology 99: 615-624) sowie das p36 oder auch als LACK bezeichnete Antigen (Gonzales-Aseguinolaza et al., Eur. J. Biochem. 259: 909-916). LACK ist ein 36 kDa Antigen von Leishmanien, das hochkonserviert ist und in allen verwandten Leishmaniaarten zu finden ist. Exprimiert wird es sowohl in der parasitären Promastigote und Amastigote, den beiden Stadien des parasitären Zyklus im Wirt. Im Mausmodell konnte gezeigt werden, daß die Induktion einer zytotoxischen Th1, auf Interferon-gamma und IL-12 Sekretion durch T-Helferzellen, Immunantwort die Überwindung einer Testinfektion fördert, während die Induktion einer IL-4 getriebenen Th2 Helferzellantwort die Infektion begünstigt. Um die Verschiebung der Immunantwort in Richtung Th1 zu fördern, setzten Gonzalo et al. Vaccinia Virus als virale Genfähre und IL-12 als Adjuvanz ein. Als Immunogen wurde p36/LACK Antigen benutzt. Verschiedene Impfregime wurden zusammengestellt. Das erfolgreichste Impfregime, Erstimmunisation mit p36 Protein, Zweitimmunisation mit rekombiantem Vaccinia Virus, kodierend für p36 und IL-12, führte zu einer durchschnittlichen Verkleinerung der Läsionen um 52% im Vergleich zu ungeimpften Mäusen. Den größten Infektionsschutz wiesen die Tiere auf, die den höchsten IgG2a Antikörpertiter hatten (Gonzalo et al., Microbes and Infection: 3 (9): 701-711).

Zum Einschleusen der die immunogenen Antigene oder Teile davon kodierenden DNA sind verschiedene chemische, physikalische und biologische Transfektionsmethoden bekannt.

Biologische Mittel zur Transfektion, sog. Genfähren, sind virale Vektoren, Plasmide oder kovalent geschlossene minimalistische DNA-Konstrukte, die im folgenden MIDGE genannt werden (MINIMALISTIC IMMUNOLOGICALLY DEFINED GENE EXPRESSION VECTORS, vgl. EP 0 914 318 B1).

Plasmide werden durch bakterielle Fermentation gewonnen. Sie enthalten neben dem gewünschten Gen auch die für ihre Vervielfältigung und Selektion notwendige DNA, üblicherweise Resistenzgene gegen bei der Fermentation verwendete Antibiotika. Diese bakterielle DNA hat den Nachteil, dass sie immunstimulatorische Sequenzen ("ISS", z.B. nicht-methylierte Cytosin-Guanin Dinukleotide, "CpG") enthalten kann. Bei Immunsuppression ist genau diese Wirkung nicht erwünscht (ausführlich beschrieben in DE 199 35 756). Bei der Verwendung von Genexpressionskonstrukten auf Basis von Plasmid-DNA besteht zudem das inhärente Risiko der Verbreitung von Antibiotikaresistenzgenen, welches insbesondere bei Schutzimpfungskampagnen nicht vertretbar ist. Aus diesem Grund ist auch die von Gurunathan et al. (J. Exp. Med., Vol 186, No. 7, (1997): 1137-1147) vorgeschlagene Methode der Vakzinierung mit eukaryotischen Expressionsvektoren, die das Leishmania-spezifische p36 LACK-Antigen enthalten, äußerst nachteilig. In der medizinischen Praxis stehen die beschriebenen Nachteile plasmidbasierter Expressionsvektoren ihrer breiten Anwendung massiv entgegen.

Die auf Grund ihrer hohen Transfektionseffizienz am häufigsten eingesetzten Genfähren sind virale Vektoren. Jedoch stehen die mit deren Einsatz verbundenen Sicherheitsrisiken einer breiten Anwendung in erheblichem Maße entgegen. Es ist bekannt, dass ein hohes Risiko einer zytotoxischen Reaktion des Wirtsorganismus auf die transfizierten Zellen besteht. So führte die Anwendung einer hohen Dosis eines Adenovirus bei einem klinischen Versuch zum Tod des Patienten; offensichtlich war die Ursache dafür eine starke Überreaktion des Immunsystems (Lehrman, 1999, Nature 401: 517-518). Weiterhin ist durch Instabilität des attenuierten Impfstammes die Rückverwandlung in einen virulenten Stamm nicht auszuschließen.

Außerdem können die viralen Bestandteile selbst immunogen wirken, was zu einer Herabsetzung ihrer Wirksamkeit durch das Immunsystems des Patienten führt.

Neben diesen Nachteilen, die durch die bisherigen Gentransfermethoden bedingt sind, ist es bisher trotz aller Bemühungen noch nicht gelungen, einen effektiven und sicheren Impfschutz gegen Leishmaniose zu entwickeln.

Aufgabe der Erfindung ist es daher, ein Mittel zur Verfügung zu stellen, welches ein sicheres, effektives und schützendes Impfen gegen Leishmaniose ermöglicht.

Die Aufgabe wird durch die Merkmale der unabhängigen Ansprüche gelöst.

Die Erfindung basiert auf der Zurverfügungsteltung eines DNA-Expressionskonstrukts zur Immunisierung von Leishmaniose-Infektionen, wobei die immunisierenden Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen, wobei die doppelstrangbildenden Einzelstränge durch kurze einzelsträngige Schlaufen aus Desoxyribonukleinsäurenukleotiden verknüpft sind, wobei die doppelstrangbildenden Einzelstränge nur aus der kodierenden Sequenz unter Kontrolle eines im zu impfenden Tier operablen Promotors und einer Terminatorsequenz besteht und zur Verstärkung der Transfektionseffizienz mit einem oder mehreren Peptiden gekoppelt ist (vgl. EP 0 941 318 B1). Ein derartiges DNA-Expressionskonstrukt zeigt überaus überraschende Wirkungen (siehe unten), die mit anderen Methoden (zitierte Impfprotokolle) nicht zu erreichen sind; insbesondere treten auch die oben beschriebenen Nachteile von eukaryotischen Expressionsvektoren bei der Vakzinierung nicht auf.

Erfindungsgemäß ist insbesondere vorgesehen, dass das immunogene p36 LACK Antigen zur Erzeugung einer Immunantwort verwendet wird. Als Genfähre wird eine linear-doppelsträngige kovalent geschlossene Expressionskassette verwendet. Diese besteht aus der kodierenden Sequenz, dem Promotor und gegebenenfalls Terminationssequenzen, so dass das Konstrukt nur die für die Expression des gewünschten Gens notwendige Information enthält (vgl. EP 0 941 318 B1) Femer ist erfindungsgemäß vorgesehen, dass das DNA-Expressionskonstrukt zur Steigerung der Transfektionseffizienz mit einem Oligopeptid kovalent verbunden ist, welches vorzugsweise eine Länge von fünf bis 25 Aminosäuren hat und mindestens die Hälfte der Aminosäuren zu der Gruppe Lysin oder Arginin gehören. Besonders bevorzugt ist eine Kemlokalisationssequenz, insbesondere
- Die ein Kem-Lokalisierungssignal (Nuclear Localization Signal = NLS) darstellende Peptidsequenz PKKKRKV (Prolin-Lysin-Lysin-Lysin-Arginin-Lysin-Valin = Seq ID 3) aus dem Simian Virus SV-40. Speziell für das SV-40-NLS wurde gezeigt, dass Proteine bis zu 465 kDa zum Zellkem gesteuert werden (Lanford et al. 1986, Cell 15; 46 (4): 575-82). Diese Fähigkeit des Peptids wurde hier durch Kopplung an DNA für die Verbesserung des Gentransfers genutzt.
- oder das elf Aminosäuren lange T-Peptidfragment (YGRKKRRQRRR = Seq ID 2) des HIV-1 Genprodukts TAT.

Weitere vorteilhafte Maßnahmen sind in den übrigen Unteransprüchen enthalten. Die Erfindung wird nachfolgend anhand von Ausführungsbeispielen und Figuren näher beschrieben und diskutiert.

Die überraschende Wirkung des erfindungsgemäßen DNA-Expressionskonstrukts und eines Arzneimittels, welches ein solches enthält, wird anhand der Darstellungen gem. der Figuren deutlich. Dabei bedeuten:
- pMOK p36: Plasmid kodierend für p36 Antigen
- Mp36-NLS: MIDGE kodierend für p36 Antigen mit NLS Peptid gekoppelt
- pMOK ctr: Konirollplasmid kodierend für HBsAg
- rWp36: rekombinanter Vaccinia Virus kodierend für p36
- Phosphat: Phosphatpuffer als Kontrolle
- Kontrolle +: Positivkontrolle, mit L. major infizierte Seren von Mäusen
- Kontrolle -: Negativkontrolle, Seren unbehandelter Mäuse

Es zeigt:

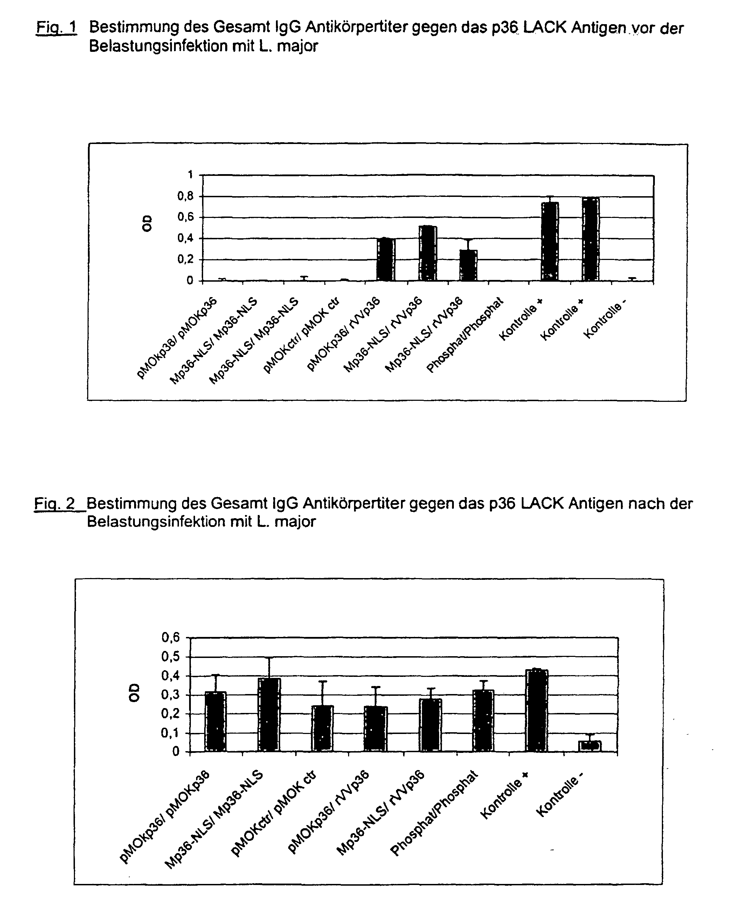

- Fig. 1:: die Bestimmung des Gesamt IgG Titer vor Belastungsinfektion mit Leishmania major Promastigoten. Nur die Impfregime, die eine Zweitimmunisierung mit rekombinantem Vaccinia Virus beinhalten, zeigen einen messbaren Anti-körpertiter.

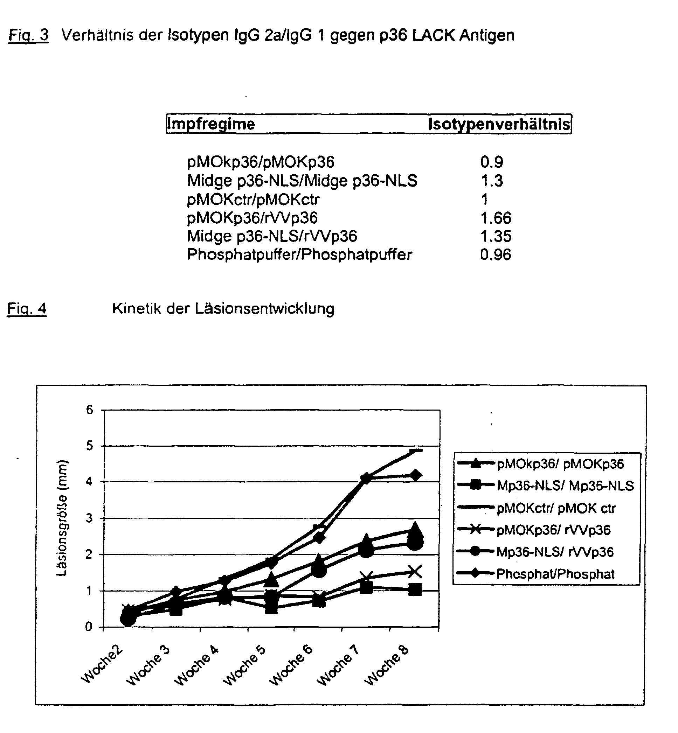

- Fig. 2:: Bestimmung des Gesamt IgG Titer nach Belastungsinfektion. Alle Impfproto- kolle zeigen eine meßbare Antikörperantwort, wobei der höchste Titer an zirkulierenden Antikörpern von MIDGE p36-NLS / MIDGE p36-NLS ausgelöst wird.
- Fig. 3:: das Verhältnis der Antikörper-Isotypen IgG 2a und IgG 1 nach Zweitimmunisierung und Belastungsinfektion mit L. major Promastigoten. Überraschenderweise lösen die mit dem NLS-Peptid gekoppelten MIDGE eine nach der Antikörper-Isotypenverteilung zytotoxische Immunantwort (Th1) aus, die sich von der durch das Regime pMOKp36/rWp36 ausgelösten nur gering unterscheidet.
- Fig.4:: die Entwicklung der Läsionen in einem Zeitraum von 8 Wochen nach der Belastungsinfektion. Dabei bewirken die Impfprotokolle basierend auf MID-GE p36-NLS/MIDGE p36-NLS und pMOK p36/ rW p36 den längsten Schutz gegen eine Infektion mit Leishmania major. Die Schutzwirkung wird durch eine deutlich verzögerte Läsionsentwicklung sichtbar. Der wirksamste und langanhaltenste Schutz wird jedoch durch MIDGE p36-NLS/MIDGE p36-NLS erreicht.

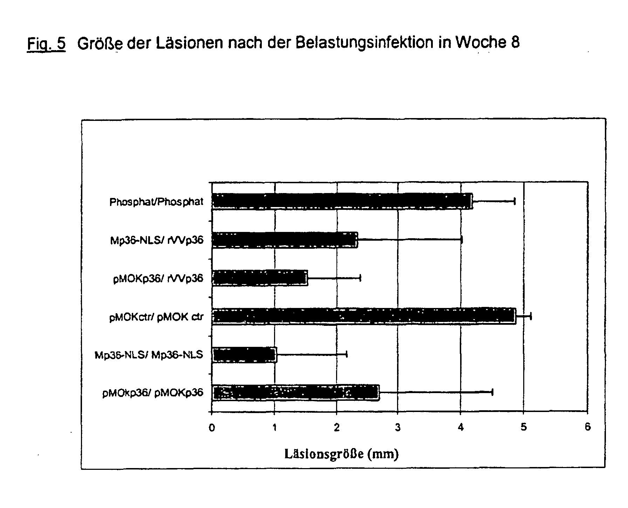

- Fig. 5:: die Größe der Läsionen nach Woche 8. In der achten Woche nach der Belastungsinfektion ist die Läsionsgröße bei den mit MIDGE p36-NLS/MIDGE p36-NLS geimpften Tieren um 80% kleiner als bei der ungeimpften Kontrollgruppe. Es konnte sogar eine 11 prozentige Steigerung des Schutzes vor L. major im Vergleich mit der mit pMOK p36/rW p36 geimpften Gruppe festgestellt werden.

Im einzelnen:

Es wurde untersucht, ob die Modifikation der minimalistischen Expressionskassetten mit Peptiden eine Änderung der Stärke oder Ausrichtung der Immunantwort zu erreichen vermag. Zur Erhöhung der Transfektionseffizienz wurde versucht, verschiedene Peptide und andere organische Moleküle an die MIDGE kovalent zu koppeln.

So ist es gelungen durch kovalente Kopplung des Kemlokalisationssignals (NLS) aus dem SV 40 Virus an für HBsAg kodierende MIDGE, einen 10- bis 15-fach erhöhten Antikörpertiter nach intramuskulärer Applikation nachzuweisen (Schirmbeck et al., J. Mol Med. 2001 Jun.79 (5-6): 343-50).

In einem Impfversuch in Mäusen wurden verschiedene Genexpressionskonstrukte, die für das Antigen p36 LACK kodierten, getestet. So wurden mit NLS Peptid gekoppelte MIDGE (MIDGE p36-NLS), Plasmid (pMOKp36) und rekombinanter Vaccinia Virus (rVVp36) eingesetzt. Um einen möglichst hohen Immunschutz zu erreichen, wurden verschiedene Impfregime formuliert. Als relevante Parameter für den klinischen Erfolg der Impfung wurde das Wachstum der infektionsbedingten Läsionen in der infizierten Hinterpfote des Tieres gemessen. Als Surrogat-Parameter wurde das Verhältnis zwischen den IgG1 und IgG2a Antikörpersubtypen bestimmt. Allgemein läßt sich feststellen, daß ein zum IgG2a verschobenes Verhältnis der Subtypen mit Schutz vor Infektion bzw. eines deutlich verlangsamstem Wachstum der Läsionen korreliert. Neben der aus dem Stand der Technik bekannten Methode der Erstimmunisierung mit Plasmid und der Zweitimmunisierung mit rekombinantem Vaccinia Virus (rW), sollte getestet werden, ob ein ähnlicher Immunschutz auch mit dem erfindungsgemäßen Arzneimittel erreicht werden kann.

Die Antikörpertiter für Gesamt IgG als Maß für die Auslösung einer Immunantwort wurden mittels ELISA bestimmt. Dabei konnten vor der Belastungsinfektion mit Leishmania major Promastigoten nur durch zwei Impfregime messbare Antikörper erzeugt werden (s. Fig. 1). In beiden Fällen wurde rekombinanter Vaccinia Virus als Zweitimmunisation (Boost) verwendet. Verschiedene Studien beweisen, dass zirkulierende Antikörper allein noch nichts über eine vermeintliche Schutzwirkung aussagen. Ein Zusammenhang zwischen zirkulierenden Antikörpern und Schutz vor Infektion kann erst nach der Belastungsinfektion gesehen werden. In Fig. 2 sind die Antikörpertiter nach der Belastungsinfektion mit L. major dargestellt. Alle Impfregime zeigen messbare Antikörpertiter, wobei der höchste mit MIDGE p36-NLS/MIDGE p36-NLS erreicht werden konnte.

Die Antikörperisotypen IgG1 und IgG2a wurden bestimmt, um einen möglichen Th2/Th1 shift in der Immunantwort nachzuweisen. Die Isotopenverteilung von Immunglobin gamma (IgG) für ein bestimmtes Antigen spiegelt die Ausprägung der gesamten Immunantwort gegen dieses Antigen wider. Dabei sind IgG -1 Subtypen charakteristisch für eine humorale Antwort, begleitet von einer verstärkten Ausschüttung von Interleukinen IL-4 und IL-10 durch aktivierte Lymphozyten, ein erhöhter Spiegel von Subtyp IgG 2a ist typisch für eine zelluläre Th1-Antwort, begleitet von erhöhter Ausschüttung von IFNγ und IL-12. Das Auftreten der Isotypen ist dabei nicht exklusiv, die relativen Titer können jedoch als Indikator für die dominante Art der entstandenen Immunantwort gewertet werden.

Gemäß Fig. 3 sind mit dem NLS Peptid gekoppelte MIDGE Vektoren in der Lage, eine zelluläre (Th1) Immunantwort auszulösen. Wie dargestellt, ist der zelluläre Arm der Immunantwort entscheidend in der Bekämpfung intrazellulärer Parasiten. Die von MIDGE p36-NLS ausgelöste Verschiebung der Th2 in Richtung Th1 Antwort, unterscheidet sich nur gering von der durch pMOKp36/rWp36 ausgelösten.

Zur Beurteilung der erreichten Schutzwirkung wurde eine Belastungsinfektion mit Leishmania major Promastigoten durchgeführt. Anhand des Größenwachstums der Läsionen in Abhängigkeit von der Zeit in Wochen wurde der Impferfolg bewertet. Dabei zeigte sich, dass die mit MIDGE p36-NLS/MIDGE p36-NLS behandelten Mäuse die vom Durchmesser kleinsten Läsionen aufwiesen, also MIDGE p36-NLS im Impfregime MIDGE p36-NLS/MIDGE p36-NLS den längsten Impfschutz bewirkt (s. Fig. 4 u. 5).

Diese Ergebnisse sind insofern sehr überraschend, da das erfindungsgemäße Arzneimittel in seiner Wirkung das derzeit im Stand der Technik als "bestes" bezeichnete Impfregime der Zweitimmunisierung (Boost) mit rekombinanten Vaccinia Virus übertrifft, und die möglichen Nebenwirkungen, die von Plasmiden und attenuierten Viren ausgehen, vermeidet (Gonzalo et al., Microbes and Infection:3 (9) :701-711). Gleichzeitig ist das erfindungsgemäße Arzneimittel vergleichbar bis besser in seiner Schutzwirkung. Die Vermeidung von potentiellen Nebenwirkungen durch Plasmide und rekombinanten Viren ist der entscheidende Vorteil des erfindungsgemäßen Arzneimittels, die Herstellung. ist einfacher und kostensparender, zudem ist das erfindungsgemäße Mittel wesentlich sicherer.

### Ausführungsbeispiele

### Beispiel 1.1: Klonierung des Plasmids pMOKp36

Vom Ausgangsplasmid pSCp36 wurden 2 Fragmente mittels PCR amplifiziert:
1. PCR ca. 800 bp;
2. PCR ca. 950 bp;

Das PCR-Produkt aus der 2. PCR wurde mit Eco31l geschnitten und das kleinere Fragment (ca. 200 bp) isoliert.

Das PCR-Produkt aus der 1. PCR wurde mit Bpil geschnitten.

Das 200 bp Fragment und das geschnittene Fragment aus der 1. PCR wurden ligiert und anschließend mit Kpnl und Sacl geschnitten und in den mit Kpnl und Sacl geschnittenen pMOK-Vektor ligiert. Das entstandene Plasmid erhielt den Namen pMOKp36 (= Seq ID 1).

### Beispiel 1.2: Kopplung der NLS Sequenz an Oligonukleotide

Die NLS Kopplung wurden wie folgt vorgenommen: das NLS Peptid mit der Sequenz PKKKRKV wurde in zwei Schritten an die ODN's gekoppelt. Zuerst wurde das modifizierte Oligonukleotid 5'-PH-dGGG AGT CCA GT xT TTC TGG AC (wobei xT für aminomodifizierte Thyminbase mit C₂ - Amminolinker steht, = ODN 1 = Seq ID 4) (0,1mM) mit sulfo-KMUS (5mM) in PBS bei Raumtemperatur (RT) aktiviert. Nach 120 min, wurde die Reaktion mit 50 mM Tris-(Hydroxymethyl)-Aminomethane gestoppt und das aktivierte ODN nach Ethanolpräzipation (300mM NaOAc pH 5.2, 5.5 mM MgCl₂, 70 % Ethanol), Zentrifugation und einmaligem Waschen mit 70% Ethanol erhalten. Das so erhaltene ODN (0,1mM) wurde in PBS gelöst und reagierte mit dem Peptid (0,2mM) für eine Stunde bei Raumtemperatur. Die Reaktion wurde durch Gelektrophorese (3%) und Ethidiumbromidfärbung überprüft. Das entstandene NLS gekoppelte ODN wurde durch HPLC aufgereinigt und zur Synthese der MIDGE p36-NLS Konstrukte verwendet.

### Beispiel 1.3: Herstellung der MIDGE p36-NLS

MIDGE sind lineare, kovalent geschlossene Expressionskassetten, die lediglich aus dem CMV Promotor, einem Intron, der entsprechenden Gensequenz und einer Polyadenylierungssequenz bestehen (vgl. EP 0 941 318 B1 ). Die Konstrukte wurden wie folgt erhalten: das unter Beispiel 1.1 beschriebene Plasmid pMOKp36 wurde mit Eco 31I vollständig verdaut. Die Ligation mit 5' phosphorylierten haarnadelförmigen Oligodesoxynukleotiden (ODN) 5'-PH-GGG AGT CCA GT XT TTC TGG AC (= ODN 1 = Seq ID 4) und 5'-AGG GGT CCA GTT TTC TGG AC-3' (= ODN 2 = Seq ID 5) durch T4 DNA Ligase in Anwesenheit von Eco 31l wurde durch Erhitzen auf 70°C gestoppt. Das resultierende Gemisch wurde konzentriert und mit Eco 31l und T7 DNA Ligase in Abwesenheit von Deoxyribonukleotid-Triphosphaten behandelt. Die Aufreinigung erfolgte durch Anionenaustauschchromatographie.

### Beispiel 1.4: p36 Antikörperbestimmung in Mäusen

MIDGE p36-NLS, pMOKp36 und rekombinanter Vaccinia Virus p36 (rW) wurden in weibliche (Balb/c) Mäuse nach folgendem Impfregime injiziert.

**Tabelle 1**

| Gruppen | Erstimmunisierung (prime) | Zweitimmunisierung (boost) |
|---|---|---|
| 1 | pMOKp36 | pMOKp36 |
| 2 | MIDGE p36-NLS | MIDGE p36-NLS |
| 3 | pMOK Kontrolle | pMOK Kontrolle |
| 4 | pMOKp36 | rWp36 |
| 5 | MIDGE p36-NLS | rWp36 |
| 7 | Phosphatpuffer | Phosphatpuffer |

Je Gruppe wurden 10 Mäuse eingesetzt.

Die verwendeten DNA Mengen betrugen für:
pMOKp36: 100µg, i.d.
MIDGE p36-NLS: 54,8µg, i.d.
rVV p36: 5x107 pfu/Maus, i.p.
und wurden in Natriumphosphatpuffer pH 7,2 gelöst, injiziert.

Nach 2 Wochen erfolgte die Zweitimmunisierung (boost) mit den entsprechenden (s. Tab. 1) DNA Konstrukten. Drei Wochen nach dem Boost erfolgte die Belastungsinfektion mit 5x10⁴ Leishmania major Promastigoten. Diese wurden den Mäusen in die rechte Hinterpfote s.c. injiziert. Der Stand der Infektion wurde wöchentlich verfolgt. Die Größe der Läsionen wurde mit einer elektronischen Schiebelehre durch Vergleich mit der linken unbehandelten Hinterpfote bestimmt.

Acht Wochen nach der Belastungsinfektion wurden von allen Mäusen die Seren gewonnen. Der Gesamt-IgG Antikörpertiter gegen das p36 Antigen und die Bestimmung der IgG 2a und IgG 1 Antikörper erfolgte mittels ELISA, wobei die Absorption in OD (Optische Dichte) bei einer Wellenlänge von λ = 406 nm bestimmt wurde.

## Patentansprüche

1. DNA-Expressionskonstrukt zur Immunisierung gegen Leishmaniose-Infektionen, **dadurch gekennzeichnet, dass** die immunisierenden Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen, wobei die doppelstrangbildenden Einzelstränge durch kurze einzelsträngige Schlaufen aus Desoxyribonukleinsäurenukleotiden verknüpft sind, wobei die doppelstrangbildenden Einzelstränge nur aus kodierender Sequenz, die wenigstens für das p36 LACK Antigen kodiert, und unter Kontrolle eines im zu impfenden Tier operablen Promotors und einer Terminatorsequenz besteht, wobei das DNA-Expressionskonstrukt zur Steigerung der Transfektionseffizienz mit einem oder mehreren Oligopeptiden kovalent verknüpft ist und das Oligopeptid aus 3 bis 30 Aminosäuren besteht, von denen mindestens die Hälfte basische Aminosäuren aus der Gruppe Arginin und Lysin kommen.

2. DNA-Expressionskonstrukt nach Anspruch 1, wobei das Expressionskonstrukt ein oder mehrere Leishmania Antigene kodiert.

3. DNA-Expressionskonstrukt nach Anspruch 1 oder 2, wobei das Oligopeptid die Aminosäuresequenz PKKKRKV (Prolin-Lysin-Lysin-Lysin-Arginin-Lysin-Valin) aufweist.

4. Verwendung des DNA-Expressionskonstrukte gemäß einem oder mehreren der Ansprüche 1 bis 3 zur Herstellung eines Impfstoffes zur Behandlung von Leishmaniose-Infektionskrankheiten.

5. Vakzine zur Behandlung von Leishmaniose-Infektionskrankheiten, enthaltend das DNA-Expressionskonstrukt nach den Ansprüchen 1 bis 4.

## Claims

1. DNA expression construct for immunisation against infections by leishmania, **characterized by** the immunising polynucleotide sequences having the form of expression constructs comprising covalently closed linear deoxyribonucleic acid molecules, which comprise a linear double stranded region, the single strands forming said double stranded region being linked by short single stranded loops of deoxyribonucleic acid nucleotides, said double strand forming single strands comprising only a coding sequence encoding at least the p36 LACK antigen under control of a promoter sequence operable in the animal that is to be vaccinated, and a terminator sequence, and where said DNA expression construct is covalently linked to one or more oligopeptides to increase the transfection efficacy, and said oligopeptide comprises 3 to 30 amino acids, half of which are basic amino acids of the group consisting of arginine and lysine.

2. DNA expression construct according to claim 1, where the expression construct encodes one or more leishmania antigens.

3. DNA expression construct according to claim 1 or 2, where the oligopeptide comprises the amino acid sequence PKKKRKV (proline - lysine - lysine - lysine - arginine - lysine - valine).

4. Use of the DNA expression construct according to one or several of claim 1 to 3 for the production of a vaccine for the treatment of leishmaniosis infectious diseases.

5. Vaccine for the treatment of leishmaniosis infectious diseases comprising the DNA expression construct according to claims 1 to 4.

## Revendications

1. Construct d'expression d'ADN destiné à immuniser contre des infections de type leishmaniose, **caractérisé en ce que** les séquences de polynucléotides immunisantes se présentent sous la forme de constructs d'expression qui se composent de molécules d'acide désoxyribonucléique linéaires fermées de façon covalente, qui présentent une zone bicaténaire linéaire, les simples brins qui forment le double brin étant liés entre eux par de courtes boucles monocaténaires de nucléotides d'acide désoxyribonucléique, les simples brins qui forment le double brin se composant uniquement d'une séquence codante qui code au moins pour l'antigène LACK/p36, et sous le contrôle d'un promoteur utilisable dans l'animal à vacciner et d'une séquence terminatrice, le construct d'expression d'ADN étant lié de façon covalente, pour augmenter l'efficacité de la transfection, à un ou plusieurs oligopeptides, et l'oligopeptide se composant de 3 à 30 acides aminés, parmi lesquels au moins la moitié des acides aminés basiques provient du groupe de l'arginine et de la lysine.

2. Construct d'expression d'ADN selon la revendication 1, dans lequel le construct d'expression code pour un ou plusieurs antigènes de Leishmania.

3. Construct d'expression d'ADN selon la revendication 1 ou 2, dans lequel l'oligopeptide présente la séquence d'acides aminés PKKKRKV (proline - lysine - lysine - lysine - arginine - lysine - valine).

4. Utilisation du construct d'expression d'ADN selon une ou plusieurs des revendications 1 à 3 pour fabriquer un vaccin destiné au traitement de maladies infectieuses de type leishmaniose.

5. Vaccin destiné au traitement de maladies infectieuses de type leishmaniose, contenant le construct d'expression d'ADN selon les revendications 1 à 4.
